# EUROPEAN PATENT SPECIFICATION

(11) **EP 2 187 216 B1**
(45) Date of publication and mention of the grant of the patent: **26.09.2012**
(21) Application number: 08827455.0
(22) Date of filing: 07.08.2008
(51) Int. Cl.: G01N 33/574, C07K 14/475, C07K 16/22

(54) **NOVEL LIVER CANCER MARKER**
NEUER LEBERKREBSMARKER
NOUVEAU MARQUEUR DE CANCER DU FOIE

(30) Priority: 10.08.2007 JP 2007209544
(43) Date of publication of application: 19.05.2010
(73) Proprietor: Hyogo College Of Medicine, Hyogo 663-8501 (JP); Otsuka Pharmaceutical Co., Ltd., Tokyo 101-8535 (JP)
(72) Inventor: NAKAMURA, Hideji, Suita-shi Osaka 565-0812 (JP); YABUUCHI, Youichi, Osaka-shi Osaka 541-0045 (JP); OHMOTO, Yasukazu, Osaka-shi Osaka 541-0045 (JP); MORI, Toyoki, Osaka-shi Osaka 541-0045 (JP); MURAGUCHI, Masahiro, Osaka-shi Osaka 541-0045 (JP); OGA, Keiko, Osaka-shi Osaka 541-0045 (JP); IWATA, Fusako, Osaka-shi Osaka 541-0045 (JP); MURATA, Kaori, Osaka-shi Osaka 541-0045 (JP)
(74) Representative: HOFFMANN EITLE
(86) International application number: PCT/JP2008/064248
(87) International publication number: WO 2009/022632

(56) References cited:
- WO-A1-2004/108964
- JP-A- 01 500 188
- JP-A- 2003 180 368
- JP-A- 2006 526 405
- HU T H ET AL: "Expression of hepatoma-derived growth factor in hepatocellular carcinoma - A novel prognostic factor", CANCER, AMERICAN CANCER SOCIETY, PHILADELPHIA, PA, US LNKD- DOI:10.1002/CNCR.11653, vol. 98, no. 7, 1 October 2003 (2003-10-01), pages 1444-1456, XP009109915, ISSN: 0008-543X
- KENYA YOSHIDA ET AL: "Hepatoma-Derived Growth Factor Is a Novel Prognostic Factor for Hepatocellular Carcinoma", ANNALS OF SURGICAL ONCOLOGY, SPRINGER-VERLAG, NE, vol. 13, no. 2, 1 February 2006 (2006-02-01), pages 159-167, XP019369776, ISSN: 1534-4681
- YOSHIDA KENYA ET AL: "Expression of hepatoma-derived growth factor in hepatocarcinogenesis.", JOURNAL OF GASTROENTEROLOGY AND HEPATOLOGY, vol. 18, no. 11, November 2003 (2003-11), pages 1293-1301, XP002591225, ISSN: 0815-9319
- DATABASE Geneseq [Online] 5 May 2005 (2005-05-05), "PRO polypeptide SEQ ID NO 130.", XP002591226, retrieved from EBI accession no. GSP:ADY14324 Database accession no. ADY14324
- HU TH: 'Expression of hepatoma-derived growth factor in hepatocellular carcinoma A novel prognostic factor' CANCER vol. 98, no. 7, 2003, pages 1444 - 1456, XP009109915
- YOSHIDA KEN'YA: 'Kangan Oyobi Kanhatsugan ni Okeru Kangan Yurai Zoshoku Inshi HDGF no Yakuwari' MED J OSAKA UNIV vol. 46, no. 1-4, 2003, pages 51 - 61, XP008130120
- KAGESHITA TOSHIRO: 'Melanoma 'Shinki Kogen ni yoru Melanoma Kessei Shindan -glypican-3 SPARC-'' THE CELL vol. 38, no. 14, 2006, pages 584 - 586, XP008130116
- MAEKAWA MASATO: 'Suigan no Soki Shindan eno Approach Suigan no Shuyo Marker' SUIZO vol. 19, no. 6, 2004, pages 579 - 583, XP003012959
- MIDORIKAWA YASUSHI: 'Genpatsusei Kansaibogan ni Oite Kohatsugen suru Glypican 3 ni yoru Kessei Shindan to FGF2 Oyobi BMP-7 no Signal Dentatsu Yokusei' JOURNAL OF JAPAN SURGICAL SOCIETY vol. 104, 2003, pages 556 - 4053-54, XP008130121
- WATANABE MASAHIKO: 'Gan no Soki Shindan Oyobi Chiryoho no Sentaku ni Yakudatsu Shuyo Marker no Kaihatsu' KOSEI RODOSHO GAN KENKYU JOSEIKIN NI YORU KENKYU HOKOKUSHU vol. 2001, 2002, pages 336 - 341, XP008130117
- HENING REN: 'Expression of hepatoma-derived growth factor is a strong prognostic predictor for patients with early-stage non-small-cell lung cancer' JOURNAL OF CLINICAL ONCOLOGY vol. 22, no. 16, 2004, pages 3230 - 3237, XP008129885
- IWASAKI TERUO: 'Hishosaibo Haigan Setsujorei ni Okeru Kangan Yurai Yoshoku Inshi HDGF no Hatsugen to sono Yogo Inshi to shite no Igi ni Tsuite' THE JOURNAL OF THE JAPANESE RESPIRATORY SOCIETY vol. 18, no. 3, 2004, pages 482 - 581
- NAKAMURA SHUJI: 'Kangan Shikkan Kesshochu no Kangan Yurai Zoshoku Inshi HDGF Nodo no Sokutei' XP008130119

### SEQUENCE LISTING

<110> HYOGO COLLEGE OF MEDICINE OTSUKA PHARMACEUTICAL CO., LTD.
<120> Novel liver cancer marker
<130> P08-90, 139898
<150> JP2007-209544
   <151> 2007-08-10
<160> 2
<170> PatentIn version 3.4
<210> 1
   <211> 240
   <212> PRT
   <213> Homo sapiens
<400> 1
   Met Ser Arg Ser Asn Arg Gln Lys Glu Tyr Lys Cys Gly Asp Leu Val
   1               5                   10                  15
   Phe Ala Lys Met Lys Gly Tyr Pro His Trp Pro Ala Arg Ile Asp Glu
               20                  25                  30
   Met Pro Glu Ala Ala Val Lys Ser Thr Ala Asn Lys Tyr Gln Val Phe
           35                  40                  45
   Phe Phe Gly Thr His Glu Thr Ala Phe Leu Gly Pro Lys Asp Leu Phe
       50                  55                  60
   Pro Tyr Glu Glu Ser Lys Glu Lys Phe Gly Lys Pro Asn Lys Arg Lys
   65                  70                  75                  80
   Gly Phe Ser Glu Gly Leu Trp Glu Ile Glu Asn Asn Pro Thr Val Lys
                   85                  90                  95
   Ala Ser Gly Tyr Gln Ser Ser Gln Lys Lys Ser Cys Val Glu Glu Pro
               100                 105                 110
   Glu Pro Glu Pro Glu Ala Ala Glu Gly Asp Gly Asp Lys Lys Gly Asn
           115                 120                 125
   Ala Glu Gly Ser Ser Asp Glu Glu Gly Lys Leu Val Ile Asp Glu Pro
       130                 135                 140
   Ala Lys Glu Lys Asn Glu Lys Gly Ala Leu Lys Arg Arg Ala Gly Asp
   145                 150                 155                 160
   Leu Leu Glu Asp Ser Pro Lys Arg Pro Lys Glu Ala Glu Asn Pro Glu
                   165                 170                 175
   Gly Glu Glu Lys Glu Ala Ala Thr Leu Glu Val Glu Arg Pro Leu Pro
               180                 185                 190
   Met Glu Val Glu Lys Asn Ser Thr Pro Ser Glu Pro Gly Ser Gly Arg
           195                 200                 205
   Gly Pro Pro Gln Glu Glu Glu Glu Glu Glu Asp Glu Glu Glu Glu Ala
       210                 215                 220
   Thr Lys Glu Asp Ala Glu Ala Pro Gly Ile Arg Asp His Glu Ser Leu
   225                 230                 235                 240
<210> 2
   <211> 2376
   <212> DNA
   <213> Homo sapiens
<400> 2
   gaggaggagt ggggaccggg cggggggtgg aggaagaggc ctcgcgcaga ggagggagca 60
   attgaatttc aaacacaaac aactcgacga gcgcgcaccc accgcgccgg agccttgccc 120
   cgatccgcgc ccgccccgtc cgtgcggcgc gcgggcggag acgccgtggc cgcgccggag 180
   ctcgggccgg gggccaccat cgaggcgggg gccgcgcgag ggccggagcg gagcggcgcc 240
   gccaccgccg cacgcgcaaa cttgggctcg cgcttcccgg cccggcgcgg agcccggggc 300
   gcccggagcc ccgccatgtc gcgatccaac cggcagaagg agtacaaatg cggggacctg 360
   gtgttcgcca agatgaaggg ctacccacac tggccggccc ggattgacga gatgcctgag 420
   gctgccgtga aatcaacagc caacaaatac caagtctttt ttttcgggac ccacgagacg 480
   gcattcctgg gccccaaaga cctcttccct tacgaggaat ccaaggagaa gtttggcaag 540
   cccaacaaga ggaaagggtt cagcgagggg ctgtgggaga tcgagaacaa ccctactgtc 600
   aaggcttccg gctatcagtc ctcccagaaa aagagctgtg tggaagagcc tgaaccagag 660
   cccgaagctg cagagggtga cggtgataag aaggggaatg cagagggcag cagcgacgag 720
   gaagggaagc tggtcattga tgagccagcc aaggagaaga acgagaaagg agcgttgaag 780
   aggagagcag gggacttgct ggaggactct cctaaacgtc ccaaggaggc agaaaaccct 840
   gaaggagagg agaaggaggc agccaccttg gaggttgaga ggccccttcc tatggaggtg 900
   gaaaagaata gcaccccctc tgagcccggc tctggccggg ggcctcccca agaggaagaa 960
   gaagaggagg atgaagagga agaggctacc aaggaagatg ctgaggcccc aggcatcaga 1020
   gatcatgaga gcctgtagcc accaatgttt caagaggagc ccccaccctg ttcctgctgc 1080
   tgtctgggtg ctactgggga aactggccat ggcctgcaaa ctgggaaccc ctttcccacc 1140
   ccaacctgct ctcctcttct actcactttt cccactccaa gcccagccca tggagattga 1200
   cctggatggg gcaggccacc tggctctcac ctctaggtcc ccatactcct atgatctgag 1260
   tcagagccat gtcttctccc tggaatgagt tgaggccact gtgttccttc cgcttggagc 1320
   tattttccag gcttctgctg ggcctgggga caactgctcc cacctcctga cacccttctc 1380
   ccactctcct aggcattctg gacctctggg ttgggatcag gggtaggaat ggaaggatgg 1440
   agcatcaaca gcagggtggg cttgtggggc ctgggagggg caatcctcaa atgcggggtg 1500
   ggggcagcac aggagggcgg cctccttctg agctcctgtc ccctgctaca cctattatcc   1560
   cagctgccta gattcaggga aagtgggaca gcttgtaggg gaggggctcc tttccataaa   1620
   tccttgatga ttgacaacac ccatttttcc ttttgccgac cccaagagtt ttgggagttg   1680
   tagttaatca tcaagagaat ttggggcttc caagttgttc gggccaagga cctgagacct   1740
   gaagggttga ctttacccat ttgggtggga gtgttgagca tctgtccccc tttagatctc   1800
   tgaagccaca aataggatgc ttgggaagac tcctagctgt cctttttcct ctccacacag   1860
   tgctcaaggc cagcttatag tcatatatat cacccagaca taaaggaaaa gacacatttt   1920
   ttaggaaatg tttttaataa aagaaaatta caaaaaaaaa ttttaaagac ccctaaccct   1980
   ttgtgtgctc tccattctgc tccttcccca tcgttgcccc catttctgag gtgcactggg   2040
   aggctcccct tctatttggg gcttgatgac tttctttttg tagctggggc tttgatgttc   2100
   cttccagtgt catttctcat ccacataccc tgacctggcc ccctcagtgt tgtcaccaga   2160
   tctgatttgt aacccactga gaggacagag agaaataagt gccctctccc accctcttcc   2220
   tactggtctc tctatgcctc tctacagtct cgtctctttt accctggccc ctctcccttg   2280
   ggctctgatg aaaaattgct gactgtagct ttggaagttt agctctgaga accgtagatg   2340
   atttcagttc taggaaaata aaacccgttg attact                              2376

## Description

### [Technical Field]

The present invention relates to a method of testing or diagnosing liver cancer.

### [Background Art]

Hepatoma-derived growth factor, HDGF, is a growth factor isolated from a culture supernatant of hepatoma cell line HuH-7. Cloning of the growth factor has been completed, and its DNA sequence has been determined (Non-Patent Document 1). Further, it is known that the growth factor is localized in the nuclear fraction, and that it has an effect of stimulating the proliferation of hepatoma cells, smooth muscle cells, fibroblast cells, and vascular endothelial cells (Non-Patent Documents 2-4). Further, HDGF is also localized in the nuclear fraction of neurons. HDGF is known to be differentially expressed in hepatoma cell lines and hepatoma tissue samples(Non-Patent Document 5, Patent Document 1). Contrary to its name, "HDGF" is widely distributed *in vivo*. Additionally, because of its nutritional factor-like action, the possibility of using HDGF for treatment and diagnosis in patients with nerve diseases has been considered.
[Non-Patent Document 1] Nakamura H. et al., JBC 269: 25143-25149 (1994)
[Non-Patent Document 2] Nakamura H. et al., Clin. Chim. Acta. 183: 273-284 (1989)
[Non-Patent Document 3] Everett A. D. et al., J. Clin. Invest. 105: 567-575 (2000)
[Non-Patent Document 4] Oliver J. A. et al., J. Clin. Invest. 102: 1208-1210(1998)
[Non-Patent Document 5] Hu T. H. et al., Cancer 98: 1444-1456 (2003)
[Patent Document 1] WO 2004/108964 A1

### [Disclosure of the Invention]

### [Object to be Achieved by the Invention]

An object of the present invention is to provide a simple method of testing or diagnosing liver cancer.

### [Means to Achieve the Object]

The inventors of the present invention produced an antibody against HDGF, and developed an HDGF measurement method based on the antibody. Further, the inventors determined, by the above-mentioned measurement method, that HDGF exists in the blood, and found that the amount of HDGF was significantly high, particularly in liver cancer patients with chronic liver disease.

The present invention provides a method of diagnosing liver cancer patients.

Item 1. An in vitro method of testing whether a patient has liver cancer, comprising detecting a liver cancer marker in a blood sample, which had been obtained from the subject, the marker being a human HDGF-derived polypeptide that binds with human HDGF-recognizing antibody, wherein the human HDGF is the amino acid sequence of SEQ ID NO: 1.

Item 2. The testing method as defined in Item 1, wherein the detection is performed by immunoassay.

Item 3. The testing method as defined in Item 2, wherein the immunoassay is an ELISA.

Item 4. The testing method as defined in Item 2, wherein the subject is a patient with chronic liver disease.

### [Effects of the Invention]

The present invention enables a diagnosis of liver cancer by measuring the HDGF in the blood of patients with chronic liver disease.

### [Brief Description of Drawings]

Fig. 1 shows a procedure for ELISA measurement of HDGF.
Fig. 2 shows a standard curve of HDGF. The detection range is 0.31 to 20 ng/ml.
Fig. 3 shows the amount of HDGF in the blood (plasma HDGF) of each patient with liver disease.

Note that HCC indicates a patient with hepatocellular carcinoma, CHC indicates a patient with hepatitis C, CHB indicates a patient with hepatitis B, and LC indicates a patient with liver cirrhosis.

### [Best Mode for Carrying out the Invention]

Liver diseases include liver cancer, viral hepatitis type B, viral hepatitis type C, cirrhosis, and other various diseases. Damage is inflicted on the liver cells themselves. Therefore, the possibility of liver disease may be determined by measuring the liver cell's contents in the blood. For example, both GOT and GPT are enzymes that are active in the liver cells. When the liver cells are destroyed due to hepatitis and the like, GOT and GPT leak into the blood. Therefore, the GOT and GPT levels in the blood are used as the indices indicative of the degree of liver damage. The GOT and GPT are valuable indices of diseases such as acute hepatitis, chronic hepatitis, etc. in which liver cells are destroyed; however, they are not appropriate as indices of liver cancer, in which the destruction of liver cells is not significant.

HDGF is present in the nuclear fraction. Because it was assumed that HDGF leaks into the blood only after the cells are destroyed, HDGF was expected to be a marker similar to GOT and GPT; however, the inventors of the present invention found, against all expectations, that HDGF, unlike GOT and GPT, is a specific liver cancer marker.

### Liver Cancer Marker

The term "human HDGF" as used in the present specification refers to a growth factor having an amino acid sequence of SEQ ID NO: 1.

The term "human HDGF-recognizing antibody" includes antibodies that recognize a partial peptide produced by decomposition of HDGF insofar as those antibodies recognize human HDGF. The antibodies may be monoclonal or polyclonal. The antibodies may also be antibody fragments insofar as they can bind to antigen. Examples of antibody fragments include scFv, Fab, F(ab)₂, Fv, etc.

The term "human HDGF-derived polypeptide" as used in the present specification includes not only polypeptides that include human HDGF itself, but also a wide range of polypeptides (including epitopes) that can be recognized by antihuman HDGF antibody, such as polypeptides that include human HDGF modified by methylation, amidation, acetylation, etc. *in vivo*, and polypeptides that are shortened by the enzymatic or chemical decomposition (specifically hydrolysis) of human HDGF.

The liver cancer marker of the present invention includes a human HDGF-derived polypeptide. Liver cancer patients show an increased amount of human HDGF-derived polypeptide in the blood. Samples used for measuring a liver cancer marker include blood, plasma, and serum.

The liver cancer marker of the present invention is expected to be usable as a higher-precision cancer marker by combining the liver cancer marker of the present invention with other conventionally known liver cancer markers such as, for example, AFP (α-fetoprotein), which is a protein produced in the liver and the yolk sac during fetal life and whose increase is observed in about 80% of patients with hepatocellular carcinoma.

### Testing Method

The liver cancer testing method of the present invention is described below in detail.

The present testing method comprises the step of detecting a liver cancer marker. The sample to be measured is blood. The sample may be either serum or plasma.

There are no particular limitations on the method of detecting or measuring a liver cancer marker insofar as the HDGF-derived polypeptide in the blood can be measured by the method. Preferable examples of such methods include commonly employed biochemical procedures, particularly procedures based on immunological principles. Preferable examples include procedures such as ELISA, Western blotting, and radioimmunoassay (RIA), which employ an antibody against a target substance to be measured. Further, when an antibody is used, the antibody may be either monoclonal or polyclonal. Alternatively, a molecule such as an aptamer, which is derived from a nucleic acid and has affinity for a target substance to be measured, may be used. Further, when contaminants are present in the sample, the target molecule is first purified, or the purity of the sample is increased to an extent where the contaminants have no effect before using the sample for the measurement. Purification methods include gel filtration, ion exchange chromatography, hydrophobic chromatography, affinity chromatography, reversed-phase chromatography, normal phase chromatography, various electrophoresis techniques, ammonium sulfate precipitation, precipitation in an organic solvent, isoelectric precipitation, etc. These methods can be used according to the situation. Needles to say, these are merely examples, and the purification method is not limited to these above-mentioned methods.

The ELISA may be either a direct method or a sandwich method when producing an ELISA kit for measuring HDGF. Measurement can be performed according to conventional methods. Further, in this case, either a monoclonal antibody or polyclonal antibody may be used. For example, in the case of a sandwich method, a primary antibody (anti-HDGF antibody) is usually immobilized on a 96-well plate. A sample derived from a living body (blood, serum, or plasma), which may be diluted, as needed, with an appropriate buffer solution, is added to the plate to allow contact with the antibody for a certain period of time, and thereby the sample is bound to the antibody. After washing the plate with an appropriate buffer solution, a labeled secondary antibody (labeled anti-HDGF antibody) is added for reaction. For example, when the labeling substance is biotin, avidin- (or streptavidin-)labeled peroxidase is added for reaction, and a suitable reactive substrate (for example, TBM) is added for color development. After a certain period of time, measurement is performed at a specific wavelength (450 nm in this case), and the colorimetric determination is thereby carried out. Peroxidase (HRP) labeling, alkaline phosphatase labeling, acid phosphatase labeling, glucose oxidase labeling, tyrosinase labeling, and the like may be used as avidin-labeled antibodies. There are no particular limitations on the substrate, insofar as the substrate is commercially available and commonly used.

Further, a Western blotting method may also be used as an immunological measurement method. In the method of the present invention, electrophoresis is performed on the sample (representative examples of electrophoresis include paper electrophoresis and isoelectric electrophoresis such as SDS-PAGE, PAGE, etc.); the sample is transferred onto a transfer membrane such as a nitrocellulose membrane, PVDF membrane, etc.; a primary antibody against a target molecule to be measured is added to the sample; and a secondary antibody bound to a labeled antibody such as a pigment particle is added to the sample, or, when an labeled enzyme is used, a substrate for the enzyme is added to the sample after the treatment, thereby allowing the target molecule to be visualized. Biotin-labeled antibody is used as a labeled antibody, to which avidin or streptavidin is bound. Or, a peroxidase (HRP)-labeled antibody, an alkaline phosphatase-labeled antibody, an acid phosphatase-labeled antibody, a glucose oxidase-labeled antibody, a tyrosinase-labeled antibody or the like is used as a secondary antibody. There are no particular limitations on the substrate, insofar as the substrate is commercially available and commonly used.

Additionally, the antibody used for measurement can be obtained according to conventional methods. For example, in the case of the production of a polyclonal antibody, warm-blooded animals such as rabbits, sheep, guinea pigs, and chickens are immunized several times with an emulsified substance usually prepared by mixing the above-mentioned target antigen (human HDGF, or its partial peptide or partial polypeptide) with Freund's complete adjuvant. A resulting anti-serum can be obtained according to conventional methods. Further, in the case of chickens, the chickens are immunized with the above-mentioned immunizing antigen several times, IgY is produced in eggs laid by the chickens, and the IgY can be obtained from the yolk of the eggs according to conventional methods.

Further, the antibody can be obtained as a monoclonal antibody. The monoclonal antibody can be obtained, for example, by immunizing mice several times with the above-mentioned immunizing antigen together with Freund's complete adjuvant to induce an antibody, isolating the resulting antibody-producing cells according to conventional methods such as a cell fusion method, and culturing the cells. The thus-obtained antibody can be further purified, as needed, according to conventional methods such as ammonium sulfate precipitation, ion exchange chromatography, affinity chromatography, etc.

### Disease and Subject to be Diagnosed

The present invention can be used for the diagnosis of patients with chronic liver disease, particularly to determine whether their chronic liver disease has progressed to liver cancer. In the case of patients with viral hepatitis or cirrhosis, cancer may be defined as the last stage of the progression of such diseases. For evaluation of the mental condition of the patients or the motivation for treatment, it is important for patients with chronic liver disease to know what pathological stage they are at.

### The Method of Diagnosis

Diagnosis of liver cancer in patients with chronic liver disease according to the present invention is made by measuring the amount of HDGF in the blood. Specifically, the present method can be performed in an ELISA kit and in a kit composition including various reagents for Western blotting. In particular, the kit includes an antibody against HDGF. Besides the antibody, the kit includes albumin such as BSA, secondary antibodies, enzyme substrates (when an enzyme is used as a label), and the like. For example, when the recognizing antibody is a biotin-labeled antibody, the measurement kit may include a peroxidase-labeled avidin as a secondary antibody. Further, the liver cancer diagnostic kit may include a substrate for the peroxidase and the like.

### [Example]

The details of the present invention are described below with reference to an Example. However, the details of the present invention are not limited to the following Example.

### Example 1

### Construction of ELISA Measurement System for HDGF

### Production of HDGF for Monoclonal Antibody-Antigen and for Standard Preparation

First of all, human HDGF gene is already known, and the gene is represented by SEQ ID NO: 2. Cloning was performed according to conventional methods. Specifically, the region from the initiation codon to the stop codon of HDGF gene was amplified by the PCR method using a human kidney cDNA library. An NdeI site was added to the 5' end of the forward primer, and an XhoI site was added to the 3' end of the reverse primer. HDGF cDNA was obtained by cloning a PCR product into pCR-Blunt vector, and confirming the DNA sequence.

Next, the HDGF cDNA of the present invention and a His-tag linker were inserted into pSecTag2 vector, and an expression vector (pSecTag2-HDGF) was thereby constructed. Further, the obtained expression vector (pSecTag2-HDGF) was introduced into CHO-K1 cells, the cells were cultured in the zeocin-containing medium under limiting dilution conditions, and resistant clones were thereby constructed. Further, the presence of HDGF in the culture supernatant was confirmed.

Thereafter, high-producing HDGF clones (CHO/HDGF clone 4) were mass-cultured, culture supernatants were collected, and antigen HDGF was purified.

Specifically, antigen HDGF was purified by Ni resin column chromatography, heparin-Sepharose column chromatography, and resource Q column HPLC to obtain immunogen and a standard preparation.

### Production of Mouse Monoclonal Antibody

Mice were immunized according to conventional methods, and a hybridoma that produces a desired antibody was selected. Specifically, mice were immunized with an emulsion prepared by mixing antigen solution with an equal volume of complete Freund's adjuvant. Immunization was performed every two weeks, for a total of 5 immunizations.

Next, splenocytes were prepared from immunized mice, and mixed with myeloma cells (P3U1) prepared in advance such that the ratio of splenocytes to P3U1 was 5:1 or 2:1. After centrifugal separation, PEG solution was added, while stirring well, to the cell sediment. After the mixture became uniform, it was kept still. The supernatant was removed after centrifugation, and the sediment was suspended in a 10% FCS/RPMI 1640 supplemented with 100 ml of HAT. The cells were seeded onto a 24-well culture plate (coaster) at 1 ml/well, and cultured at 37°C in 5% CO₂ to allow cell fusion.

Positive hybridomas were cloned in the following manner.

A culture supernatant was removed from each well one week to 10 days after cell fusion, and antibody screening was performed. The culture supernatant was reacted on a 96-well plate (NUNC) to which HDGF antigen was immobilized to check for the presence of antibodies by ELISA.

For the production of ascites, mice were intraperitoneally injected with pristane (2,6,10,14-etramethylpentadecan) at 0.5 ml/body at least 3 days in advance of cell injection. Hybridoma cells were suspended in 1.5 ml of PBS, and 3 mice were each injected intraperitoneally with 0.5 ml of hybridoma cells. Ascites was collected from the mice when the abdomens of the mice injected with the cells were largest.

Monoclonal antibody was purified from ascites by applying the ascites to protein A column, and the concentration thereof was verified by checking the purity by SDS-PAGE.

### Production of Rabbit Polyclonal Antibody

HDGF antigen solution was mixed with complete Freund's adjuvant to prepare an emulsion, and rabbits were intradermally immunized on the back at several sites (1 mL/site).

Immunization was performed every two weeks. Blood was partially collected from the earlobe one week after three immunizations. The antibody titer of the serum was checked by ELISA, and four more immunizations were repeated. All of the blood was collected from the carotid artery one week after the final immunization (blood collection with heparin). The blood was subjected to centrifugal separation to obtain plasma.

Note that for the production of a polyclonal antibody that uses C-terminal peptide as an antigen, a peptide having a Cys at the N-terminal of the C-terminal peptide (amino acids at positions 231 to 240 from the N-terminal) was synthesized, purified, and coupled to KLH as the antigen (KLH-C-APGIRDHESL).

### Establishment of ELISA

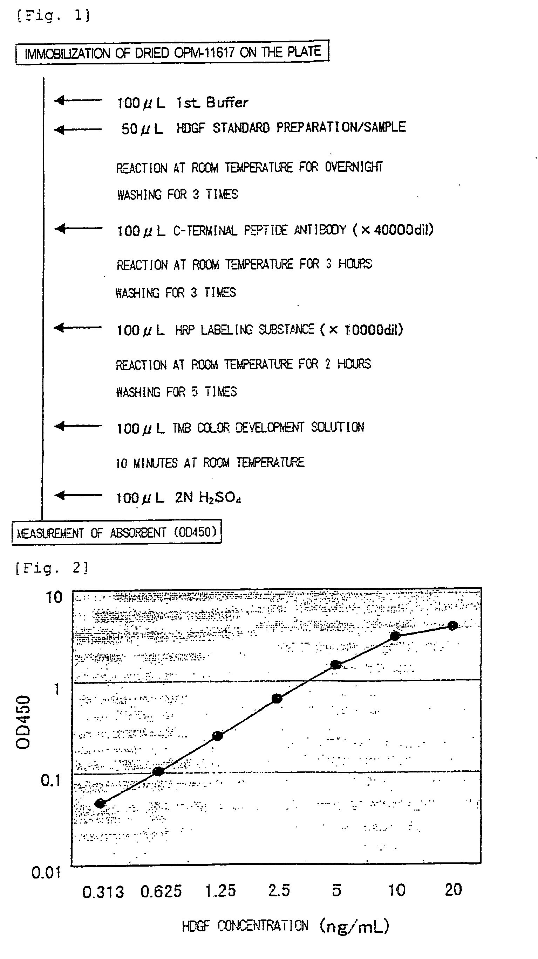

For ELISA for HDGF, monoclonal antibody OPM-11617 was diluted to 5 µg/mL in 0.1 M of NaCO₃, immobilized in each well, and blocked with 0.1% BSA/PBS. 0.1% BSA/PBS/0.05% Tween-20 was used as a first buffer, which was placed in a 96-well plate in advance. The standard HDGF was diluted using 20 mg/mL of BSA/PBS. 7 two-fold serial dilutions from 20 ng/ml were prepared as standard preparations having different concentrations, and a blank was used to determine the calibration curve. A volume of 50 µL of standard preparation and sample was added to the well and allowed to react at room temperature for 24 hours. After washing the well, C-terminal antibody (100 µL) diluted 40,000-fold by 0.1% BSA/PBS/0.05% Tween-20 was added to the well, and allowed to react at room temperature for 2 hours. Further, the plate was washed, and HRP-labeled avidin diluted 10,000 times was added to each well and allowed to react at room temperature for 2 hours. After washing the plate, TMB solution was added to the plate and allowed to react at room temperature for 10 minutes. The reaction was stopped with 2N sulfuric acid. The absorbance was set to 450 and measured at the wavelength. Fig. 1 shows a procedure for measurement.

Fig. 2 shows a calibration curve of HDGF. The measurement range is from 0.31 ng/ml to 20 ng/mL. The measurement of HDGF in cell culture was made possible.

### Measurement of HDGF in Serum of Patients with Chronic Liver Disease

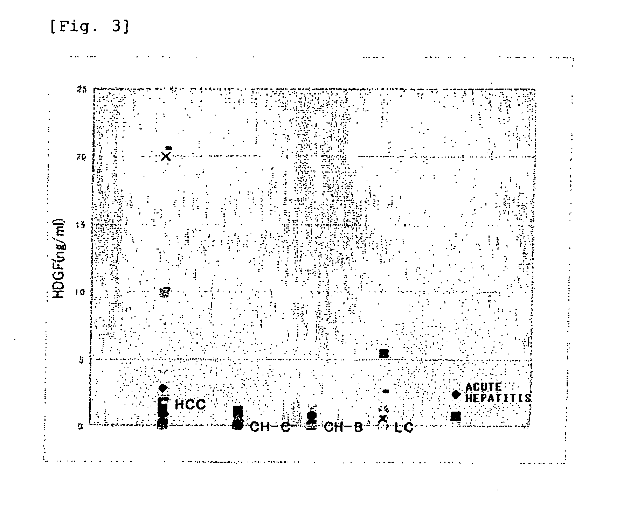

Blood was collected from patients with liver cancer (61 people), viral hepatitis type C (61 people), viral hepatitis type B (9 people), cirrhosis (55 people), and acute hepatitis (3 people) during fasting, and the value of HDGF in plasma was measured using the above-described established ELISA system. The results show that the amount of HDGF is significantly high in plasma from patients with liver cancer (Fig. 3, Table 1).

**[Table 1]**

| | Liver Cancer | Hepatitis C | Hepatitis B | Liver Cirrhosis | Acute Hepatitis |
|---|---|---|---|---|---|
| Negative | 25 | 54 | 4 | 40 | 1 |
| Positive | 36 | 7 | 5 | 15 | 2 |
| Total/240 | 61 | 61 | 9 | 55 | 3 |

## Claims

1. An in vitro method of testing whether a patient has liver cancer, comprising detecting a liver cancer marker in a blood sample, which had been obtained from the subject, the marker being a human HDGF-derived polypeptide that binds with human HDGF-recognizing antibody, wherein the human HDGF is the amino acid sequence of SEQ ID NO: 1.

2. The testing method as defined in claim 1, wherein the detection is performed by immunoassay.

3. The testing method as defined in claim 2, wherein the immunoassay is an ELISA.

4. The testing method as defined in claim 2, wherein the subject is a patient with chronic liver disease.

## Patentansprüche

1. In vitro-Verfahren zum Testen, ob ein Patient Leberkrebs hat, umfassend das Nachweisen eines Leberkrebsmarkers in einer Blutprobe, welche von dem Patienten erhalten wurde, wobei der Marker ein humanes von HDGF abgeleitetes Polypeptid ist, welches humanen HDGFerkennenden Antikörper bindet, wobei das humane HDGF die Aminosäuresequenz der SEQ ID NO: 1 ist.

2. Testverfahren gemäß Anspruch 1, wobei der Nachweis mittels einem Immunoassay durchgeführt wird.

3. Testverfahren gemäß Anspruch 2, wobei der Immunoassay ein ELISA ist.

4. Testverfahren gemäß Anspruch 2, wobei der Patient einer mit chronischer Leberkrankheit ist.

## Revendications

1. Procédé de test *in vitro* servant à déterminer si un patient a un cancer du foie, comprenant la détection d'un marqueur de cancer du foie dans un échantillon sanguin, qui a été prélevé sur le sujet, le marqueur étant un polypeptide dérivant du HDGF humain qui se lie à un anticorps reconnaissant le HDGF humain, dans lequel le HDGF humain est la séquence d'acides aminés de SEQ ID N° : 1.

2. Procédé de test selon la revendication 1, dans lequel la détection est effectuée au moyen d'un immunoessai.

3. Procédé de test selon la revendication 2, dans lequel l'immunoessai est un ELISA.

4. Procédé de test selon la revendication 2, dans lequel le sujet est un patient souffrant d'une maladie hépatique chronique.
